# EUROPEAN PATENT SPECIFICATION

(11) **EP 1 433 463 B1**
(45) Date of publication and mention of the grant of the patent: **22.09.2010**
(21) Application number: 03029218.9
(22) Date of filing: 18.12.2003
(51) Int. Cl.: A61K 8/64, A61K 8/97, A61Q 5/00, A61Q 5/02, A61Q 19/00, A61Q 19/08

(54) **Use of algal proteins in cosmetics**
Verwendung von Algenproteinen in Kosmetika
Utilisation de protéines d'algues dans des produits cosmétiques

(30) Priority: 26.12.2002 JP 2002376271
(43) Date of publication of application: 30.06.2004
(73) Proprietor: SHIRAKO Co., Ltd., Tokyo, 134-8502 (JP)
(72) Inventor: Hagino, Hiroshi, Tokyo, 12-0022 (JP); Saito, Masanobu, Chiba-shi, Chiba-ken 262-0044 (JP)
(74) Representative: HOFFMANN EITLE

(56) References cited:
- EP-A- 0 619 112
- EP-A- 0 699 431
- DE-A- 1 617 340
- FR-A- 1 125 342
- FR-A- 2 728 467
- FR-A- 2 792 832
- US-A- 5 916 577
- PATENT ABSTRACTS OF JAPAN vol. 0142, no. 54 (C-0724), 31 May 1990 (1990-05-31) & JP 2 072108 A (YOSHIO TANAKA), 12 March 1990 (1990-03-12)
- PATENT ABSTRACTS OF JAPAN vol. 0140, no. 96 (C-0692), 22 February 1990 (1990-02-22) & JP 1 305011 A (SANSHO SEIYAKU CO LTD), 8 December 1989 (1989-12-08)
- DATABASE WPI Section Ch, Week 198534 Derwent Publications Ltd., London, GB; Class D21, AN 1985-208921 XP002275777 & SU 1 138 161 A (BIOTECH RES INST ET AL.) 7 February 1985 (1985-02-07)
- PATENT ABSTRACTS OF JAPAN vol. 0102, no. 62 (C-371), 6 September 1986 (1986-09-06) & JP 61 087614 A (DAIICHI SEIMO KK), 6 May 1986 (1986-05-06)
- DATABASE CHEMICAL ABSTRACTS 12 May 1984 (1984-05-12), XP0002074633 retrieved from STN Database accession no. 88:41555 & JP 52 125635 A (CHLORELLA SUN CO.) 21 October 1977 (1977-10-21)

## Description

### Technical Field

The present invention relates to the cosmetic use of algal proteins and their hydrolyzates, that is, algal peptides.

### Background Art

Algae contain various nutriments such as vitamins, minerals, dietary fibers etc., and are noticeable for keeping one-self in good health by improvement in eating habits. In recent years, algae are not only eaten as such, but are also proposed to be used as products keeping one-self in good health as a result of extensive study paying attention to their physiological activity. The present inventors have also made study while paying attention particularly to the biological activity of algae of the genus Porphyra and wakame seaweeds, and as a result, they found that proteins from algae of the genus Porphyra and wakame seaweeds, or peptides as hydrolyzates of the proteins, have physiological activities such as a blood pressure-dropping action, a hepatic function-improving action, a lipid metabolism-improving action, a peripheral blood vessel-expanding action and a blood viscosity-reducing action, and they developed application thereof to pharmaceutical preparations, health foods etc. (see, for example, Japanese Patent No. 3272621 and JP-A 2000-157226).

The physiological activity of algae is utilized not only by oral ingestion in the form of pharmaceutical preparations, health foods etc., but also by external application onto the skin. For example, polysaccharides obtained from algae have suitable viscosity and simultaneously maintain moisture, and thus their application to cosmetics etc. has been attempted. Further, a substance having an ultraviolet absorbing action or a substance having an inhibitory action on synthesis of melanin has been found in some algae, thus making the cosmetic effect of the algae more noticeable.

However, there have never been any examples wherein algal proteins or peptides having diverse physiological activities as described above are utilized as major components in cosmetics. In only a few reports, it is proposed that fermentation products, by yeasts or lactic acid bacteria, of solutions obtained by decomposition of algae (that is, laver) of the genus Porphyra with a fibrinolytic enzyme or an acid are used as cosmetics (see, for example, JP-A 61-87614), but their cosmetic effect is estimated to be attributable to supply of nutrients from hydrolysates of seaweeds of the genus Porphyra containing various components to the skin, and their active component is not specified. Because fermentation processing can induce antigenicity, there is also concern about their safety.

FR 2 792 832 discloses the use of heat shock proteins isolated from algae, for example Chlorella, in the context of skin aging.

JP 02072108 discloses the use of amino acids derived from Chlorella for cosmetics. The amino acids are supposed to be effective in moistening the skin.

JP 01305011 relates to a complex algae extract derived from wakame seaweeds, and its use for a cosmetic having excellent moisture retention.

FR 2 728 467 relates to a protein extract derived from e.g. Chlorella and cosmetic uses thereof.

FR 1 125 342 also relates to cosmetic uses of Chlorella cultures.

SU 19833630251 discloses an increased lipolytic activity and content in the skin associated with Chlorella protein hydrolysate.

JP 610876614 describes cosmetics effective to supply nutrients to the skin, comprising algal decomposition products, e.g. proteins and amino acid decomposition products.

EP 0 619 112 discloses a shampoo comprising Spirulina, characterized by lack of eye irritation.

JP 76-42109 describes cosmetics for skin care containing extracts of Chlorella and refers to their nutrient effects.

DE 1 617 340 describes cosmetic products comprising powdered algae, wherein amino acids are liberated from the algae.

RU 2044770 discusses cosmetics comprising extraction products from Chlorella micro-algae.

On the other hand, proteins derived from animals or plants, or peptides thereof obtained by hydrolysis, have affinity for the hair or skin, and are thus used widely in cosmetics. Specifically, elastin, collagen, casein, and proteins or peptides of wheat, soybeans etc. are utilized as cosmetics. However, animal-derived peptides are deliberately not used in recent years because of problems such as mad cow disease. In peptides of wheat, soybeans etc., on one hand, there is concern about antigenicity. There are algal proteins as a source of promising proteins, but there is no example where the algal proteins, or peptides obtained by hydrolysis of the proteins, are utilized.

### Summary of Invention

This invention was made to cope with these circumstances, and the object of this invention is to provide the use of naturally occurring algae as the starting material, for cosmetics exhibiting protective and cosmetic effects on the skin and hair.

The present inventors studied the biological activity of algal proteins or their hydrolyzates i.e. algal peptides, and as a result, they found that these substances have affinity for the skin and hair to exhibit an excellent cosmetic effect based on the affinity, and this invention was thereby arrived at

That is, this invention relates to the cosmetic use of algal proteins or their hydrolyzates that are peptides.

### Disclosure of Invention

Hereinafter, the constitution of this invention is described in more detail.

The starting materials of algae proteins and peptides used in this invention are seaweeds of the genus Porphyra as edible algae, wakame seaweeds and spirulinahaving a relatively high content of proteins.

Now, the method of producing algal proteins from the starting algae is described. From the algae, proteins are obtained by solvent extraction. Because cell walls of the algae are relatively strong, it is effective in this case to use techniques wherein the algae are previously disrupted or the algae are milled with a solvent at the time of extraction, or the cells walls are decomposed with a fibrinolytic enzyme. From this extract, the proteins are then fractionated and purified. These techniques include precipitation of the proteins with an organic solvent such as ethanol or with ammonium sulfate, ion-exchanger absorption, precipitation with polyethylene glycol, isoelectric precipitation and separation through a membrane, and these techniques may be simultaneously used.

The algal proteins thus obtained have strong affinity for the skin and hair, to confer moisture retention on the skin and hair and to improve the feel of the skin and hair. Cosmetics compounded with the algal proteins, as compared with cosmetics not compounded therewith, were confirmed to significantly improve the feel in use.

Now, production of peptides derived from algae is described. The peptides derived from algae are obtained from the algal proteins by enzyme decomposition, acid or alkali hydrolysis, or treatment such as heating extraction under pressure. The starting materials of the algal peptides are the above-described algal proteins, or the peptides may be removed after directly hydrolyzing the algae.

In the case of enzyme decomposition, generally used enzymes having a protease activity, such as pepsin, pancreatin, papain, Prolaser (Amano Pharmaceutical Co., Ltd.), Samoase (Yamato Kasei Co., Ltd.), Sumizyme AP, Sumizyme MP, Sumizyme FP (Shin-Nippon Kagaku Kogyo Co., Ltd.), etc. can be used alone or as a mixture thereof. The concentration of the enzyme added for the reaction, the pH of the reaction solution, the reaction temperature, and other conditions may be selected such that the conditions are optimum for the enzyme used.

For acid or alkali decomposition, an organic or inorganic acid or an alkali may be used, and preferably the pH in acid hydrolysis is in the range of 1 to 4, and the pH in alkali hydrolysis in the range of pH 8 to 13, and the decomposition temperature and time are suitably established. In these hydrolysis methods, acid or alkali hydrolysis and/or enzyme decomposition may be used.

The peptides obtained in this manner were confirmed to have stronger affinity for the skin and hair than that of the proteins.

Not only the physical properties of the proteins or peptides but uses thereof as cosmetics are also varied depending on their molecular weight, and it is preferable that those having a desired molecular weight are fractionated by molecular-weight fractionation, or conditions for decomposition to give peptides having a desired molecular weight are established to prepare algal proteins or peptides adapted to intended uses. In either case, there are a wide variety of methods for extraction and decomposition of the proteins, and a suitable method can be selected for production.

The algal proteins or peptides may be used in the form of their derivatives obtained by reactions such as esterification, cationization or acylation depending on the intended use.

When the algal proteins and algal peptides are used for various purposes, they can be used in a contaminated state, but preferably components other than the proteins or peptides are removed by ultrafiltration, treatment with an adsorbent, ethanol precipitation, ionexchange chromatography, or other suitable methods. If necessary, the algal proteins and peptides can be used after being dried alone or together with excipients such as starch, dextrin etc. or other cosmetic materials by a method such as spray drying, lyophilization or the like.

Both the algal proteins and algal peptides exhibit high affinity for the skin and hair and simultaneously exhibit an excellent cosmetic effect, and cosmetics compounded therewith are recognized to significantly improve the feel in use. The algal proteins and peptides can be incorporated in the range of 0.0005% (W/W) to 28% (W/W), particularly preferably in the range of 0.01% (W/W) to 10% (W/W), based on the cosmetics. The algal proteins and/or algal peptides may be used. However, the peptides can be added more easily to cosmetics because they are more highly soluble in water and highly stable to heat, ultraviolet rays, acids, alkalis, organic solvents etc.

The cosmetics can be used in various forms such as aqueous cosmetics, emulsified cosmetics of water/oil or oil/water type, and oily cosmetics. As used herein, the cosmetics include, for example, skin care and hair care products such as face lotion, milky lotion, cream, ointment, shaving mousse, an oil pack, shampoo, rinse, treatment, hair tonic, hairdressing, a hair restorer, a solution for permanent wave, a hair coloring agent and body soap, or bath agents (which may be in any forms of liquid, powder, granule, solid etc.).

Other examples include sanitary articles, wet tissue paper, paper towel or cotton, disinfecting or therapeutic pharmaceutical preparations applied to scratches, cuts, burns, cracks, inflammations, eczema, rashes, acnes and rough skins, gauze or adhesive tapes impregnated therewith, eye care agents, inhalations, oral cavity washes, gargles, toothpastes etc.

The algal proteins and algal peptides can be used as such, but if necessary, fats and oils, wax, mineral oils, fatty acids, alcohols, polyvalent alcohols, esters, metal soaps, gum, saccharides, water-soluble polymers, surfactants, various vitamins, various amino acids, various additives derived from materials in plants or animals, metabolites from microbial cultures, and various components used in a pharmaceutical or non-pharmaceutical preparation, cosmetics and a bath agent shown below can be arbitrarily selected and used in easily applicable product forms.

### Best Mode for Carrying Out the Invention

Hereinafter, embodiments of the invention are described.

### Production Example 1

Laver proteins of seaweed of the genus Porphyra were prepared in the following manner.

10 L water was added to 2 kg laver powder (10 to 50 meshes) which was then mixed and milled with an automatic mortar at room temperature for 1 hour. Then, the milled material was separated into laver extraction residues (precipitates) and a supernatant by centrifugation (3000 r.p.m.) for 20 minutes. After ethanol was added to the resulting supernatant, the sample was left at -20°C for 12 hours to precipitate protein components, and further separated into precipitates and a supernatant by centrifugation with a centrifuge (3000 r.p.m.) for 20 minutes, to give 400 g water-soluble laver proteins as precipitates.

### Production Example 2

Laver peptides were prepared in the following manner.

50 kg dry laver was suspended in 950 L hot water heated at 95°C and then boiled for 1 hour, and the broth was removed. Thereafter, 950 L water at 50°C was added thereto and adjusted to pH 2.0 with sulfuric acid, and 2 kg pepsin (Amano Pharmaceutical Co., Ltd.) was added thereto and reacted at 50°C for 24 hours under stirring. The resulting decomposed solution was adjusted to pH 5.0 with 1 N NaOH and kept at 50°C for 10 minutes to inactivate the pepsin. Then, extraction residues were removed by centrifugation (14000 r.p.m. for 20 minutes), and the supernatant was purified by a filter press with diatomaceous earth as filter aid, then concentrated under reduced pressure and spray-dried to give a pepsin-digested laver product.

1 kg of the pepsin-digested laver product was dissolved in distilled water, applied onto a Dowex-50 (H⁺) column, φ50 cm×200 cm (Bio-Rad) previously equilibrated with hydrochloric acid, then the column was washed with 120 L distilled water, and the adsorbed peptide was eluted with 2 N ammonia water. After the ammonia was removed with an evaporator, the sample was lyophilized to give 385 g of 99% laver peptides.

0.1 g of the peptides thus obtained were dissolved in 10 ml deionized water, and 50 µl of the aliquot was applied onto GS-320HQ (φ7.6×300 mm, SHOWA DENKO K.K.), then eluted with 6 M guanidine hydrochloride as eluent at a rate of 0.8 ml/min. and detected at 220 nm to determine its molecular weight distribution. The molecular weight distribution of the laver peptides, determined from a calibration curve of substances of known molecular weights analyzed under the same conditions as above, is shown in Table 1. 2% of the peptides had a molecular weight in the range of 10,000 or more, 47% in the range of 10000 to 5000, 32% in the range of 5000 to 1000, and 19% in the range of 1000 or less.

**Table 1**

| M.W. 10000 or more | M.W 10000 to 5000 | M.W 5000 to 1000 | M.W. 1000 or less |
|---|---|---|---|
| 2% | 47% | 32% | 19% |

### Production Example 3

Proteins of wakame seaweed was prepared by the following method.

Dry wakame seaweed prepared by drying cultivated wakame seaweed was finely divided into powder of 35-mesh size with a high-speed pulverizer. 20 g of the powder was muddled in 400 ml distilled water and milled with a wet mill. Then, the sample was centrifuged (3000 r.p.m. for 20 minutes) to give 100 ml wakame protein-containing solution. 800 ml ethanol was added to the wakame protein-containing solution and left at -20°C for 12 hours to precipitate the proteins. The sample was then centrifuged (3000 r.p.m. for 20 minutes) to give precipitates. The precipitates were air-dried to give 2 g water-soluble wakame proteins.

### Production Example 4

Low-molecular laver peptides were prepared by using acid hydrolysis and enzyme decomposition, as shown below.

100 g laver proteins were dissolved in 1 L of 1 N hydrochloric acid and decomposed with the acid by heating at 100°C for 2 hours. Then, the solution was adjusted to pH 6.0 with sodium hydrochloride, and after 5 g Sumizyme FP (Shin-Nippon Kagaku Kogyo Co., Ltd.) was added thereto, the solution was decomposed at 40°C for 8 hours. The decomposed solution was heated at 100°C for 20 minutes to inactivate the enzyme, followed by removing substances of molecular weights of 100000 or more through an ultrafiltration membrane for the fractionation molecular weight of 100000.

The resulting peptides were measured for their molecular-weight distribution by the method described in Production Example 2. The molecular weight distribution of the laver peptides, determined from a calibration curve of substances of known molecular weights analyzed under the same conditions as above, is shown in Table 2. 1% of the peptides had a molecular weight in the range of 10,000 or more, 15% in the range of 10000 to 5000, 22% in the range of 5000 to 1000, and 62% in the range of 1000 or less.

**Table 2**

| M.W. 10000 or more | M.W. 10000 to 5000 | M.W. 5000 to 1000 | M.W 1000 or less |
|---|---|---|---|
| 1% | 15% | 22% | 62% |

### Production Example 5 (Reference example)

Chlorella peptides were prepared by the following method.

50 g dry powder of Chlorella was dissolved in 1 L of 0.5 N sodium hydroxide and decomposed with the alkali by heating at 80°C for 5 hours. Then, the sample was neutralized with hydrochloric acid and applied onto a Dowex-50 (H⁺) column (φ10 cm×65 cm) previously equilibrated with hydrochloric acid, then the column was washed with 5 L distilled water, and the adsorbed peptides were eluted with 2 N ammonia water. After the ammonia was removed with an evaporator, the sample was lyophilized to give 21 g of 99% chlorella peptides.

### Production Example 6

Spirulina peptides were prepared by the following method.

20 L of 1 N sodium hydroxide was added to 1 kg Spirulina powder and stirred at ordinary temperatures for 12 hours. Then, Spirulina extraction residues (precipitates) were removed by centrifugation (3000 r.p.m., 40 minutes), whereby a Spirulina alkali-soluble protein solution was obtained. Ethanol was added at a final concentration of 80% to the Spirulina alkali-soluble protein solution and then left at -20°C for 12 hours, to precipitate its protein components which were then separated into precipitates and a supernatant by a centrifuge (3000 r.p.m., 20 minutes) to give proteins as the precipitates.

Then, the protein components were dispersed in 2 L phosphate buffer (pH 7.7), and 2 g thermolysin was added thereto and reacted at 40°C for 8 hours, to hydrolyze the proteins. Then, the reaction solution was applied onto a Dowex-50 (H⁺) column (φ10 cm×65 cm) previously equilibrated with hydrochloric acid, then the column was washed with 5 L distilled water, and the adsorbed peptides were eluted with 2 N ammonia water. After the ammonia was removed with an evaporator, the sample was lyophilized to give 459 g of 99% Spirulina peptides.

### Production Example 7

The low-molecular laver peptides obtained in Production Example 4 were used to prepare laver peptide ethyl ester.

10 g of the low-molecular peptides were introduced into a 200-ml three-necked flask, and after 100 g ethanol was added thereto, the sample was heated to the boiling point of ethanol under stirring, and the ethanol was refluxed with a condenser. A hydrochloride acid gas was blown at a rate of 15 ml/min. for 10 minutes into the reaction solution which was further refluxed for additional 2 hours to finish the reaction. The reaction solution was cooled under stirring and neutralized by adding sodium hydroxide little by little to the reaction solution. Then, the reaction solution was decolorized by adding 10 g activated carbon and concentrated under reduced pressure to give 10% low-molecular peptide ethyl ester.

### Production Example 8

The laver proteins obtained in Example 1 were used to prepare an acylated laver peptide derivative.

100 g of the laver proteins obtained in Example 1, 5 g sodium hydroxide and 5 L water were introduced into a reaction device capable of reducing the pressure therein, and then mixed and subjected to hydrolysis at 50 to 60°C for 6 hours under stirring. Then, the reaction mixture was cooled to 40°C and then left for 1 hour under reduced pressure, to give laver peptides having a molecular weight of about 400 with a solids content of 48%. Then, 20 g 2-propanol and 8 g propylene glycol were added thereto, and while the mixture was kept at pH 10 with 1 N sodium hydroxide, 25 g lauric acid chloride was added dropwise thereto at 50°C. Thereafter, the mixture was left at 40 to 50°C for 1 hour, then adjusted to pH 2 with hydrochloric acid, washed with water, and degassed for 2 hours under reduced pressure. Thereafter, the reaction mixture was neutralized with sodium hydroxide to give an acylated laver peptide derivative with a solids content of 42%.

### Production Example 9

The wakame proteins obtained in Production Example 3 were used to prepare a cationized wakame peptide derivative.

10 g of the wakame proteins were dipped in 600 g aqueous solution containing 0.02 M Tris buffer, and then 6 ml 2-mercaptoethanol was added as a reducing agent. The sample was then adjusted to pH 8.5 with 1 N hydrochloric acid and subjected to reduction reaction in a nitrogen stream at room temperature for 36 hours. Then, 2 g glycidyltrimethyl ammonium chloride was added to the reaction system and stirred at 50°C for 6 hours, to give an aqueous solution of a cationized wakame peptide derivative. Then, low-molecular impurities such as the reducing agent were removed by ultrafiltration, and the filtrate was concentrated and lyophilized to give 7 g cationized wakame peptide.

### Production Example 10 (Reference example)

The low-molecular laver peptides obtained in Production Example 4 were used to prepare a silylated laver peptide derivative.

The low-molecular laver peptides in Production Example 4 were adjusted to pH 9.5 by adding 20% aqueous sodium hydroxide dropwise thereto, and then heated to 55°C. Separately, 2.6 g silane coupling agent TSL8390 (Toshiba Silicone) was dissolved at a concentration of 15% in water and then adjusted to pH 3.5 with dilute hydrochloric acid and stirred at 50°C for 15 minutes to convert its methoxy group by hydrolysis into a hydroxyl group. Then, the low-molecular laver peptide solution was stirred at 55°C, during which a solution of the silane coupling agent having the hydroxyl group after conversion was added dropwise to the peptide solution over 30 minutes. Thereafter, the mixture was further stirred at 55°C for 7 hours to complete the reaction. After the reaction was finished, the degree of introduction of a silyl functional group into amino-form nitrogen of the low-molecular laver peptides was determined by measuring the amino-form nitrogen, indicating that the degree of the silyl functional group was 42%. The reaction solution was neutralized with dilute hydrochloric acid, then desalted in an electric dialyzer, adjusted to pH 6.5 and concentrated to regulate its concentration, to give 75g of 20% aqueous silylated low-molecular laver peptide derivative solution.

### Formulation Example 1. Skin lotion

The laver peptides in Production Example 2 were used to produce a lotion with the following formulation.

| | |
|---|---|
| Sorbitol | 2.0 (weight-%) |
| 1,3-Butylene glycol | 2.0 |
| Polyethylene glycol 1000 | 1.0 |
| Polyoxyethylene oleyl ether (25E.O.) | 2.0 |
| Ethanol | 10.0 |
| Dry powder of the laver peptides | 1.0 |
| Plant extract (aloe) | 3.0 |
| Preservative | 0.3 |
| pH regulating agent | suitable amount |
| Purified water | the balance |

The control composition was the same as the above composition except that the laver peptides were not added.

The above composition and the control composition were examined for feel in use by a panel of 15 examiners. The results are shown in Table 3. The skin lotion using the laver peptides was found to exhibit higher "fitness to the skin", "gloss of the skin", and "moist feel" than the control.

**Table 3**

| | Formulation 1 | Control |
|---|---|---|
| Easy fitness | 12 | 7 |
| Gloss of the skin | 15 | 4 |
| Moist feel | 14 | 5 |

### Formulation Example 2. Milky lotion

The laver proteins in Production Example 1 were used to produce a milky lotion with the following formulation.

| | |
|---|---|
| Squalane | 3.0 (weight-%) |
| Vaseline | 1.0 |
| Stearyl alcohol | 0.3 |
| Sorbitan monostearate | 1.5 |
| Polyoxyethylene (20) sorbitan monooleate | 3.0 |
| 1,3-Butylene glycol | 5.0 |
| Dry powder of the laver proteins | 0.5 |
| Water-soluble collagen | 0.5 |
| Methylparaben | 0.4 |
| Purified water | 84.8 |

### Formulation Example 3. Skin cream

The wakame proteins in Production Example 3 were used to produce skin cream with the following formulation.

| | |
|---|---|
| Squalene | 20.0 (weight-%) |
| Beeswax | 5.0 |
| Refined jojoba oil | 5.0 |
| Glycerine monostearate | 2.0 |
| Sorbitan monostearate | 2.0 |
| Polyoxyethylene (20) sorbitan monostearate | 2.0 |
| Glycerine | 5.0 |
| Dry powder of the wakame proteins | 0.3 |
| Methylparaben | 0.2 |
| Purified water | 58.5 |

### Formulation Example 4. Body soap

The laver proteins in Production Example 1 were used to produce body soap with the following formulation.

| | |
|---|---|
| Potassium laurate | 15.0 (weight-%) |
| Potassium myristate | 5.0 |
| Propylene glycol | 5.0 |
| Dry powder of the laver proteins | 3.0 |
| Propylparaben | 0.4 |
| pH regulating agent | suitable amount |
| Purified water | the balance |

### Formulation Example 5. Shampoo

The laver peptide ethyl ester in Production Example 7 was used to produce a shampoo with the following formulation.

| | |
|---|---|
| Lauryl sulfate triethanol amine | 5.0 (weight-%) |
| Polyoxyethylene lauryl ether Na sulfate | 12.0 |
| 1,3-Butylene glycol | 4.0 |
| Lauric diethanol amide | 2.0 |
| Solution of the laver peptide ethyl ester | 2.0 |
| Disodium edetoate | 0.1 |
| Hydroxypropyl chitosan | 0.4 |
| Preservative | 0.3 |
| Perfume | 0.1 |
| Purified water | 74.1 |

### Formulation Example 6. Rinse

The low-molecular laver peptides in Production Example 4 and the chlorella peptides in Production Example 5 were used to produce a rinse with the following formulation.

| | |
|---|---|
| Stearyl trimethyl ammonium chloride | 2.0 (weight-%) |
| Cetostearyl alcohol | 2.0 |
| Polyoxyethylene lanolin ether | 3.0 |
| Propylene glycol | 5.0 |
| Dry powder of the low-molecular laver peptides | 1.5 |
| Dry powder of the Chlorella peptides | 0.5 |
| Propylparaben | 0.4 |
| pH adjusting agent | suitable |
| Purified water | the balance |

### Formulation Example 7. Bath agent

The laver peptides in Production Example 2 were used to produce a bath agent with the following formulation.

| | |
|---|---|
| Sodium bicarbonate | 56.0 (weight-%) |
| Anhydrous sodium sulfate | 30.0 |
| Borax | 2.0 |
| Dried powder of the laver peptides | 10.0 |
| Plant extract powder (chamomile & touki [Japanese Angelica root]) | 2.0 |

### Formulation Example 8. Treatment agent

The laver peptides in Production Example 2 and the Spirulina peptides in Production Example 6 were used to produce two kinds of bath agents with the following formulation.

| | |
|---|---|
| Stearic acid | 15.0 (weight-%) |
| Laver peptides | 10.0 |
| Spirulina peptides | 5.0 |
| Propylene glycol | 5.0 |
| 1,3-Butylene glycol | 5.0 |
| Ethyl alcohol | 2.0 |
| Phenoxy ethanol | 0.5 |
| Stearyl trimethyl ammonium chloride | 0.3 |
| Methylparaben | 0.3 |
| Propylp araben | 0.1 |
| Perfume | 0.05 |
| Purified water | 56.75 |

The control composition was the same as the above composition except that the laver peptides and Spirulina peptides were not added.

The above composition and the control composition were examined for feel in use by a panel of 20 examiners. The results are shown in Table 4. The treatment agent using the laver peptides and Spirulina peptides was found to exhibit higher effects on "moist feel", "gloss on the hair", and "smooth combing and good touch" than the control.

**Table 4**

| | Formulation 8 | Control |
|---|---|---|
| Moist feel | 18 | 11 |
| Fluffy feel | 12 | 12 |
| Gloss | 17 | 10 |
| Smooth combing | 16 | 9 |
| Good touch | 19 | 8 |

### Formulation Example 9. Hair styling gel

The acylated laver peptide derivative in Production Example 8 was used to produce a shampoo with the following formulation.

| | |
|---|---|
| Solution of the acylated laver peptide derivative | 10.0 (weight-%) |
| Carboxyvinyl polymer | 1.0 |
| Polyoxyethylene cetyl ether | 0.5 |
| 1,3-Butylene glycol | 3.0 |
| 2-Amino-2-methyl propanol | 0.3 |
| Ethanol | 10.0 |
| Propylparaben | 0.1 |
| Perfume | 0.5 |
| Purified water | 74.6 |

### Formulation Example 10. Shampoo

The cationized wakame peptide derivative in Production Example 9 was used to produce a shampoo with the following formulation.

| | |
|---|---|
| Cationized wakame peptide derivative | 5.0 (weight-%) |
| Polyoxyethylene cetyl ether | 2.0 |
| Hydroxyethyl cellulose | 1.0 |
| Propylene glycol | 5.0 |
| Perfume | 0.5 |
| Purified water | 86.5 |

### Formulation Example 11. (Reference Example) Treatment mousse

The silylated laver peptide derivative in Production Example 10 was used to produce a treatment base, and the treatment base and an LPG gas were introduced in a weight ratio of 8 : 2 into a pressure container to prepare a treatment mousse.

| | |
|---|---|
| Silylated laver peptide derivative | 15.0 (weight-%) |
| Polyoxyethylene lauryl ether | 1.0 |
| Ethanol | 8.0 |
| Perfume | 0.5 |
| Purified water | 75.5 |

### Industrial Applicability

According to the present invention, there can be provided cosmetics conferring gloss and moisture on the skin with high affinity and suitable convergence for the skin or conferring smoothness, moisture and gloss on the hair with excellent adsorption and permeation.

## Claims

1. Use of algal proteins or derivatives thereof for conferring moisture retention to the skin and hair, wherein the algae are seaweeds of the genus Porphyra, wakame seaweeds or Spirulina, and wherein the derivatives are algal proteins which were esterified, cationized or acylated.

2. Use of peptides obtainable by hydrolysis of algal proteins, or esterified, cationized or acylated derivatives of these peptides, wherein the algae are seaweeds of the genus Porphyra, wakame seaweeds or Spirulina for conferring moisture retention to the skin and hair.

3. The use according to claim 2, wherein the hydrolysis is hydrolysis with a protease and/or an acid or an alkali.

4. The use according to any one of claims 1 to 3, wherein said algal proteins, peptides or derivatives thereof are comprised in a cosmetic.

## Patentansprüche

1. Verwendung von Algenproteinen oder Derivaten davon, um der Haut und dem Haar eine Feuchtigkeitsspeicherung zu verleihen, wobei die Algen Meeresalgen der Gattung Porphyra, Wakame-Meeresalgen oder Spirulina sind, und wobei die Derivate Algenproteine sind, die verestert, kationisiert oder acyliert wurden.

2. Verwendung von Peptiden, die durch Hydrolyse von Algenproteinen erhältlich sind, oder veresterten, kationisierten oder acylierten Derivaten dieser Peptide, wobei die Algen Meeresalgen der Gattung Porphyra, Wakame-Meeresalgen oder Spirulina sind, um der Haut und dem Haar eine Feuchtigkeitsspeicherung zu verleihen.

3. Verwendung gemäß Anspruch 2, wobei die Hydrolyse eine Hydrolyse mit einer Protease und/oder einer Säure oder einem Alkali ist.

4. Verwendung gemäß einem der Ansprüche 1 bis 3, wobei die Algenproteine, Peptide oder Derivate davon in einem Kosmetikum enthalten sind.

## Revendications

1. Utilisation de protéines d'algues ou de dérivés de celles-ci pour conférer une rétention d'humidité à la peau et aux cheveux, où les algues sont des algues du genre Porphyra, des algues wakame ou Spirulina, et où les dérivés sont des protéines d'algues qui ont été estérifiées, cationisées ou acylées.

2. Utilisation de peptides pouvant être obtenus par hydrolyse de protéines d'algues, ou de dérivés estérifiés, cationisés ou acylés de ces peptides, où les algues sont des algues du genre Porphyra, des algues wakame ou Spirulina, pour conférer une rétention d'humidité à la peau et aux cheveux.

3. Utilisation selon la revendication 2 où l'hydrolyse est une hydrolyse avec une protéase et/ou un acide ou un alcali.

4. Utilisation selon l'une quelconque des revendications 1 à 3 où lesdites protéines d'algues, peptides ou dérivés de celles-ci sont comprises dans un cosmétique.
